# EUROPEAN PATENT APPLICATION

(11) **EP 4 631 960 A1**
(43) Date of publication of application: **15.10.2025**
(21) Application number: 22967971.7
(22) Date of filing: 16.12.2022
(51) Int. Cl.: C07K 7/06, A61P 19/00, A61K 38/00

(54) **PEPTIDE HAVING INHIBITORY ACTIVITY AGAINST OSTEOCLAST DIFFERENTIATION, AND USE THEREOF**

(30) Priority: 09.12.2022 KR 20220171932
(71) Applicant: Caregen Co., Ltd., Anyang-si, Gyeonggi-do 14119 (KR)
(72) Inventor: CHUNG, Yong Ji, Anyang-si, Gyeonggi-do 14119 (KR); KIM, Eun Mi, Anyang-si, Gyeonggi-do 14119 (KR); YEON, Jeong Tae, Anyang-si, Gyeonggi-do 14119 (KR); CHO, Hyun Joo, Anyang-si, Gyeonggi-do 14119 (KR)
(74) Representative: Potter Clarkson
(86) International application number: PCT/KR2022/020544
(87) International publication number: WO 2024/122715

(57) **Abstract**

The present application relates to a peptide having osteoclast differentiation inhibitory activity, and uses thereof, and provides a peptide consisting of an amino acid sequence of SEQ ID NO: 1 or SEQ ID NO: 2, and a pharmaceutical composition for preventing or treating bone diseases, including the peptide as an active ingredient.

## Description

### Technical Field

The present application relates to a peptide having inhibitory activity against osteoclast differentiation, and a use thereof.

### Background Art

Bones support the soft tissues and body weight of the human body and surround the internal organs, protecting them from external impact. Additionally, they are one of the important parts of the human body that not only structurally support muscles and organs, but also store calcium and other essential minerals in the body, such as phosphorus and magnesium. Therefore, the bones of adults that have finished growing do not stop until the day they die, and they maintain balance by continuously and dynamically repeating the process of creation and absorption, removing old bone and replacing it with new bone, and this is called bone remodeling. Bone turnover, the process of removing old bone and replacing it with new bone, is essential to repairing microscopic bone damage caused by growth and stress and maintaining proper bone function.

Meanwhile, it is known that two types of cells are largely involved in bone remodeling. One of the two cells is an osteoblast, which creates bone, and the other is an osteoclast, which destroys bone. Osteoblasts produce receptor activator of nuclear factor-κB ligand (RANKL) and its decoy receptor, osteoprotegerin (OPG). When RANKL binds to RANK, a receptor on the surface of osteoclast progenitor cells, osteoclast progenitor cells mature into osteoclasts, resulting in bone resorption. However, when OPG binds to RANKL, the binding between RANKL and RANK is blocked, inhibiting the formation of osteoclasts and preventing excessive bone resorption. The absorption or destruction of old bone is accomplished by osteoclasts, cells that originate from blood cells (hematopoietic stem cells), which make holes in the bone and release small amounts of calcium into the bloodstream, which is used to maintain body functions. Meanwhile, osteoblasts, which are produced from bone cells, rebuild the skeleton by filling the holes with collagen and covering them with calcium and phosphorus deposits (hydroxyapatite) to create strong new bone. When the osteoclastic rate and osteogenic rate are in balance, effective bone density may be maintained, and when their balance is disrupted, many diseases may occur, especially osteoporosis and diseases related to bone damage caused by bone metastasis of cancer cells.

Bisphosphonate-based therapeutics such as Fosamax (ingredient name: alendronate) and Actonel (ingredient name: risedronate) are used to treat bone damage caused by osteoporosis and bone metastasis of cancer cells. Most of these bisphosphonate agents work by slowing or stopping bone loss by weakening the function of osteoclasts, which destroy bone, and inducing their apoptosis. However, there have been reports of cases of osteonecrosis of the jaw, severe atrial fibrillation, bone or joint weakness, or musculoskeletal pain in patients taking bisphosphonates.

Against this technological background, multifaceted research is being conducted to discover new substances for treating bone diseases (Korean Patent Publication No. 10-2016-0024463), but it is still insufficient.

### Disclosure of Invention

### Technical Problem

One aspect is to provide a peptide consisting of an amino acid sequence of SEQ ID NO: 1 or SEQ ID NO: 2.

Another aspect is to provide a pharmaceutical composition for preventing or treating bone disease including the above peptide as an active ingredient.

Other objectives and advantages of the present application will become apparent from the following detailed description, together with the appended claims and drawings. Anything not set forth herein may be readily recognized and inferred by one skilled in the art or similar art and is hereby omitted.

### Solution to Problem

Each of the descriptions and embodiments disclosed in the present application may be applied to other descriptions and embodiments. In other words, all combinations of the various elements disclosed in the present application fall within the scope of the present application. Furthermore, the scope of the present application is not to be considered limited by the specific description set forth below.

One aspect provides a peptide consisting of an amino acid sequence of SEQ ID NO: 1 or SEQ ID NO: 2.

As used herein, the term "peptide" may refer to a linear molecule formed by combining amino acid residues to each other by peptide bonds. The peptides may be prepared according to chemical synthesis methods known in the art, in particular solid phase synthesis technique or liquid phase synthesis technique (US Registered Patent No. 5,516,891). As a result of diligent efforts to develop a peptide with biologically effective activity, the inventors of the present disclosure have identified a peptide consisting of an amino acid sequence of SEQ ID NO: 1 or SEQ ID NO: 2. Herein, the biologically effective activity may be any one or more selected from the following properties: (a) inhibition of osteoclast differentiation; (b) inhibition of expression of nuclear factor-activated T cells cytoplasmic 1 (NFATc1), or c-Fos; (c) inhibition of expression of tartrate-resistant acid phosphatase (TRAP), osteoclast-associated receptor (OSCAR), cathepsin K (CTSK), or dendritic cell-specific transmembrane protein (DC-STAMP); and (d) inhibition of expression of ATPase H+ Transporting V0 Subunit D2 (Atp6v0d2). Therefore, the peptide may be utilized for uses for the prevention or treatment of bone diseases by inhibiting differentiation of osteoclasts.

The peptide may have a protecting group bonded to the N- or C-terminus of the peptide to acquire chemical stability, enhanced pharmacological properties (half-life, absorption, titer, effect, etc.), altered specificity (for example, broad spectrum of biological activity), or reduced antigenicity. In an embodiment, an N-terminus of the peptide may be bonded with any one protecting group selected from the group consisting of an acetyl group, a fluoreonylmethoxycarbonyl group, a formyl group, a palmitoyl group, myristyl group, stearyl group, butoxycarbonyl group, allyloxycarbonyl group, and polyethylene glycol (PEG); and/or the C-terminus of the peptide may be bonded with any one protecting group selected from the group consisting of an amino group(-NH₂), a tertiary alkyl group, and an azide (-NHNH₂). Additionally, the peptide may optionally further include a targeting sequence, a tag, labeled residues, or an amino acid sequence prepared for the specific purpose of increasing the half-life or peptide stability.

The above peptide is artificially synthesized, or non-naturally occurring or engineered, and the "non-naturally occurring or engineered" refers to a state that is not in its natural state, but is created by artificial modification. Here, the artificial modification may include artificially synthesizing an amino acid sequence by mimicking a plurality of amino acid structures, or manipulating it to acquire chemical stability, enhanced pharmacological properties, altered specificity, or reduced antigenicity as described above.

As used herein, the term "stability" may refer to not only in vivo stability, which protects the peptide from attack by proteolytic enzymes in vivo, but also storage stability (for example, room temperature storage stability).

Another aspect provides a pharmaceutical composition for preventing or treating bone disease, including, as an active ingredient, a peptide consisting of an amino acid sequence of SEQ ID NO: 1 or SEQ ID NO: 2.

In the above description of the peptide or composition, the terms or elements referred to are the same as those already mentioned.

As used herein, the term "prevention" refers to any act of inhibiting or delaying the onset of a disease by administering the composition.

As used herein, the term "treatment" refers to any form of treatment that provides an effect to a subject suffering from a disease or at risk of developing a disease, including improving the condition of the subject (for example, one or more symptoms), delaying the progression of the disease, delaying the onset of symptoms, or slowing the progression of symptoms, etc. Therefore, the above "treatment" and "prevention" are not intended to refer to cure or complete elimination of symptoms.

The above "individual" refers to a person in need of treatment of a disease, and more specifically, a mammal, such as a human or non-human primate, mouse, dog, cat, horse, and bovine, etc.

As used herein, the term "bone disease" may include, for example, a disease, disorder or condition associated with RANKL-mediated signaling, including a disease, disorder or condition associated with the regulation of bone formation and resorption, as well as pathological conditions involving bone loss, including osteopenia, osteoporosis and osteolysis. The above bone disease may be, but is not limited to, osteoporosis, osteogenesis imperfecta, osteomalacia, osteonecrosis, rickets, osteomyelitis, alveolar bone loss, Paget's disease of bone, hypercalcemia, primary hyperparathyroidism, myeloma, bone loss in rheumatoid arthritis, bone loss due to cancer, fibrous dysplasia, aplastic bone disease, metabolic bone disease or age-related loss of bone mass.

Meanwhile, although prior functional peptides have effective biological activity, they have shown disadvantages such as not being able to effectively enter target tissue or cell due to the size of the peptide itself, or disappearing from the body in a short period of time due to their short half-life. In contrast, a pharmaceutical composition according to an example includes, as an active ingredient, a peptide consisting of about 10 or less amino acids, and accordingly, the penetration rate, etc., of the active ingredient into the skin is very excellent, and may provide an effective bone disease treatment effect, for example, when administered topically.

According to an embodiment, the peptide may inhibit the expression of NFATc1 and c-Fos, which are transcription factors for osteoclast differentiation induced by RANKL, as well as the expression of TRAP, OSCAR, CTSK, DC-STAMP and Atp6v0d2, which are downstream signal transducers associated with differentiation, thereby inhibiting the function of osteoclasts, therefore, the peptide may be utilized as an active ingredient of a pharmaceutical composition for treating bone diseases (J Bone Metab 2014;21:233-241 http://dx.doi.org/10.11005/jbm.2014.21.4.233 pISSN 2287-6375 eISSN 2287-7029).

The pharmaceutical composition may include, a pharmaceutically effective amount of the peptide; and/or a pharmaceutically acceptable carrier, but is not limited thereto.

As used herein, the term "pharmaceutically effective amount" may refer to an amount sufficient to achieve the cartilage regeneration efficacy of the pharmaceutical composition.

The weight ratio between the peptide and a pharmaceutically acceptable carrier may be, for example, 500:1 to 1:500, for instance, the weight ratio may be 450:1 to 1:450, 400:1 to 1:400, 350:1 to 1:350, 300:1 to 1:300, 250:1 to 1:250, 200: 1 to 1:200, 150:1 to 1:150, 100:1 to 1:100, 80:1 to 1:80, 60:1 to 1:60, 40:1 to 1:40, 20:1 to 1:20, 10:1 to 1:10, 8:1 to 1:8, 6:1 to 1:6, 4:1 to 1:4, or 2:1 to 1:2, but is not limited thereto.

The pharmaceutically acceptable carrier is commonly used in formulations and include, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, and mineral oil, etc., but is not limited thereto. Suitable pharmaceutically acceptable carriers and formulations are described in detail in Remington's Pharmaceutical Sciences (19th ed., 1995).

In addition to the above ingredients, the pharmaceutical composition may additionally include, lubricants, humectant, sweetener, flavoring agent, emulsifier, suspending agent, preservative, etc., but is not limited thereto.

The pharmaceutical composition may be administered orally or parenterally, preferably parenterally, and if parenterally administered, may be administered by, but not limited to, intramuscular injection, intravenous injection, subcutaneous injection, intraperitoneal injection, topical administration, transdermal administration, etc.

The dosage of the pharmaceutical composition may be, but is not limited to, 0.0001 to 1000 ug (micrograms), 0.001 to 1000 ug, 0.01 to 1000 ug, 0.1 to 1000 ug, or 1.0 to 1000 ug per day, and may be varied by factors such as method of formulation, mode of administration, patient age, weight, sex, medical condition, food, time of administration, route of administration, rate of excretion, and response sensitivity.

The pharmaceutical composition may be formulated, with a pharmaceutically acceptable carrier and/or excipient, according to a method that could be readily practiced by a person skilled in the art to which the present disclosure belongs, and the composition may be prepared into a unit dosage form or may be contained in a multi-dose container.

The formulation may be in the form of a solution, suspension or emulsion in an oil or aqueous medium, or in the form of an ointment, cream, gel, transdermal absorbent, cataplasma, patch, paste, extract, powder, granule, tablet or capsule, and may additionally include a dispersant and/or a stabilizer.

The peptide may be incorporated into a nanosome or a nanoparticle to further improve skin penetration or stability issues. For example, the nanosome may be prepared by a microfluidizer using lecithin as a raw material, and may be incorporated in lecithin particles. Any method of preparing the nanosome may be used as long as it is known. The size of the nanosome particle is preferably 30 to 200 nm. If the size of the nanosome particle is less than 30 nm, skin penetration may progress very quickly, resulting in skin side effects, and if the size is greater than 200 nm, skin penetration may not be easy, making it difficult to achieve the effect of using the nanosome structure.

Another aspect provides a method of preventing or treating a bone disease, including administering to a subject a composition including, as an active ingredient, a peptide consisting of an amino acid sequence of SEQ ID NO: 1 or SEQ ID NO: 2.

In the above description of the peptide, composition, etc., the terms or elements referred to are the same as those already mentioned.

As used herein, the terms "applying," "administering," and "applying" are used interchangeably and may refer to causing at least partial localization of a composition according to an example to a desired site, or to placing a composition according to an example into an individual by route of administration.

Another aspect provides a cosmetic composition, including as an active ingredient a peptide consisting of an amino acid sequence of SEQ ID NO: 1 or SEQ ID NO: 2.

In the above description of the peptide, composition, etc., the terms or elements referred to are the same as those already mentioned.

The cosmetic composition may include, a cosmetically effective amount of the peptide; and/or a cosmetically acceptable carrier, but is not limited thereto.

### Advantageous Effects of Invention

According to the peptide according to an aspect, osteoclast differentiation may be inhibited by inhibiting the osteoclast differentiation transcription factor induced by RANKL and the downstream signal transducer associated with differentiation.

According to the peptide according to an aspect, it may be applied to prevent or treat bone disease by inhibiting differentiation of osteoclasts.

### Brief Description of Drawings

FIG. 1 shows the results of examining the level of osteoclast differentiation under a microscope after treating RAW264.7 cells with RANKL together with Peptide-1 to induce differentiation into osteoclasts.
FIG. 2 shows the results of examining the level of osteoclast differentiation under a microscope after treating RAW264.7 cells with RANKL together with Peptide-2 to induce differentiation into osteoclasts.
FIG. 3 shows the results of confirming CCK-8 activity after treating RAW264.7 cells with Peptide-2.
FIG. 4 shows the results of evaluating TRAP activity after treating RAW264.7 cells with RANKL together with Peptide-1 to induce differentiation into osteoclasts.
FIG. 5 shows the results of evaluating TRAP activity after treating RAW264.7 cells with RANKL together with Peptide-2 to induce differentiation into osteoclasts.
FIG. 6 shows the results of confirming the inhibition of mRNA expression of NFATc1 or c-Fos after treating RAW264.7 cells with RANKL together with Peptide-1 to induce differentiation into osteoclasts.
FIG. 7 shows the results of confirming the inhibition of mRNA expression of NFATc1 or c-Fos at (A) 24 hours or (B) 48 hours, after treating RAW264.7 cells with RANKL together with Peptide-2 to induce differentiation into osteoclasts.
FIG. 8 shows the results of confirming the inhibition of protein expression of NFATc1 after treating RAW264.7 cells with RANKL together with Peptide-1 to induce differentiation into osteoclasts.
FIG. 9 shows the results of confirming the inhibition of protein expression of NFATc1 or c-Fos at (A) 24 hours or (B) 48 hours, after treating RAW264.7 cells with RANKL together with Peptide-2 to induce differentiation into osteoclasts.
FIG. 10 shows the results of confirming the inhibition of mRNA expression of TRAP, OSCAR, CTSK, or DC-STAMP after treating RAW264.7 cells with RANKL together with Peptide-1 to induce differentiation into osteoclasts.
FIG. 11 shows the results of confirming the inhibition of mRNA expression of TRAP, OSCAR, CTSK, DC-STAMP, or Atp6v0d2 at (A) 24 hours or (B) 48 hours, after treating RAW264.7 cells with RANKL together with Peptide-2 to induce differentiation into osteoclasts.
FIG. 12 shows the results of confirming the inhibition of mRNA expression of c-Fos or NFATc1 at (A) 3 hours or (B) 6 hours, after treatment of osteoclast precursor cells induced by RANKL with Peptide-2.
FIG. 13 shows the results of confirming the inhibition of protein expression of c-Fos or NFATc1 at (A) 3 hours or (B) 6 hours, after treatment of osteoclast precursor cells induced by RANKL with Peptide-2.
FIG. 14 shows the results of confirming the inhibition of mRNA expression of TRAP, OSCAR, CTSK, DC-STAMP, or Atp6v0d2 at (A) 3 hours or (B) 6 hours, after treatment of osteoclast precursor cells induced by RANKL with Peptide-2.

### Mode for the Invention

The following examples will be described in greater detail below. However, these examples are provided for illustrative purposes only and the scope of the present disclosure is not limited to these examples.

### Example 1: Synthesis of Peptide

Using an automatic peptide synthesizer (Milligen 9050, Millipore, USA), peptides (Peptide-1 or Peptide-2) having amino acid sequences of SEQ ID NO: 1 or SEQ ID NO: 2 as shown in Table 1 below were synthesized, and these synthesized peptides were purified using C18 reverse-phase high-performance liquid chromatography (HPLC) (Waters Associates, USA). The column used was an ACQUITY UPLC BEH300 C18 (2.1 mm X 100 mm, 1.7 µm, Waters Co, USA).

**[Table 1]**

| **Name** | **Amino acid sequence (N terminus - > C terminus)** | **SEQ ID NO:** |
|---|---|---|
| Peptide-1 | EGSNSRTI | 1 |
| Peptide-2 | IIAPTGYYGN | 2 |

### Example 2. Confirmation of Effect of Inhibiting Differentiation Into Osteoclasts

The aim was to determine whether treatment with Peptide-1 or Peptide-2 inhibited osteoclast differentiation during the process of inducing differentiation into osteoclasts by treating RAW264.7 cells, a precursor osteoclast cell line, with RANKL.

### 2-1. TRAP Staining and Cytotoxicity Evaluation

RAW264.7 cells were seeded in 96-well plates at a density of 1.7 x 10³ cells/well and cultured in DMEM for 24 hours. After 24 hours, RANKL and Peptide-1 or Peptide-2 were treated at different concentrations, and differentiation into osteoclasts was induced for 4 days. Afterwards, TRAP staining was performed using the acid phosphatase kit from Sigma Aldrich. A fixation buffer was added thereto, followed by a reaction for 30 seconds and washed with distilled water. A staining solution was added at 200 µl per well, followed by a reaction at 37 °C for 30 minutes, and then washed three times with distilled water. After drying for one day, the staining results were checked under a microscope.

In addition, to further confirm the cytotoxicity of Peptide-2, RAW264.7 cells cultured in DMEM for 24 hours were treated with the peptide at each concentration in the same manner as described above. Then, after 3 days, CCK-8 (Dojindo, CCK-8 kit) solution was added to 1/10 volume of the culture medium, diluted, and incubated for 2 hours. Thereafter, the culture medium was sampled and CCK-8 activity was confirmed at a wavelength of 450 nm using a microplate reader.

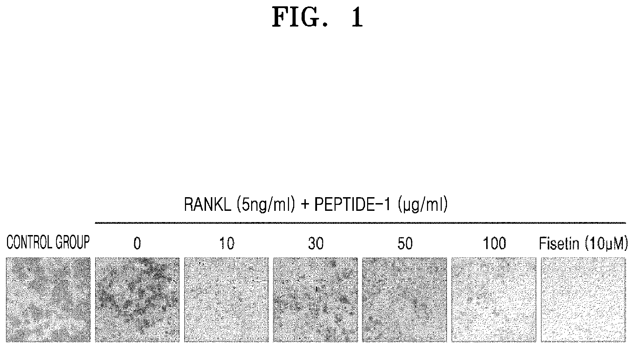

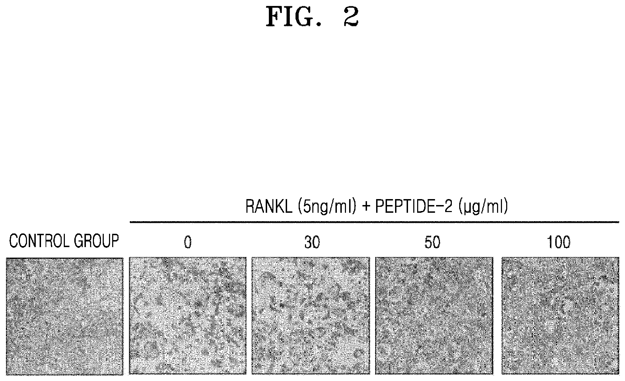

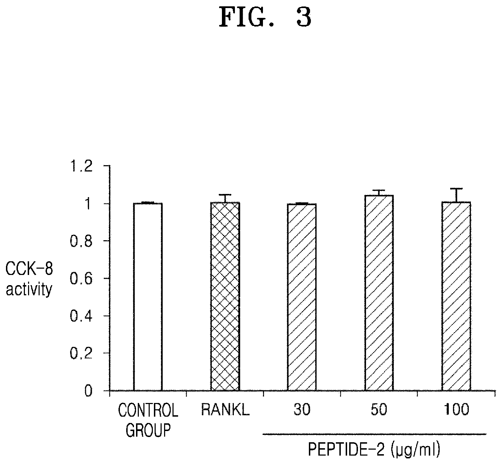

As a result, as shown in FIG. 1 and FIG. 2, it was confirmed that TRAP expression was significantly reduced in the group treated with Peptide-1 or Peptide-2, compared to the control group in which osteoclast differentiation was induced by RANKL treatment and TRAP expression was increased. Additionally, as shown in FIG. 3, it was found that Peptide-2 did not exhibit cytotoxicity.

### 2-2. TRAP Activity Evaluation

In the same manner as described above, RAW264.7 cells cultured in DMEM for 24 hours were treated with RANKL and Peptide-1 or Peptide-2 at different concentrations, and differentiation into osteoclasts was induced for 4 days. Afterwards, 100 ul of TRAP activation solution [TRAP buffer 15 ml (0.1 sodium citrate + 50 µM sodium tartrate, pH 5.0) and 4-Nitrophenly phosphate disodium salt hexahydrate (sigma, 1 tablet mix)] was added thereto, incubated at 37 °C for 1 hour, and treated with 10 ul of 2N NaOH. Afterwards, absorbance at a wavelength of 405 nm was measured using a microplate reader and quantified.

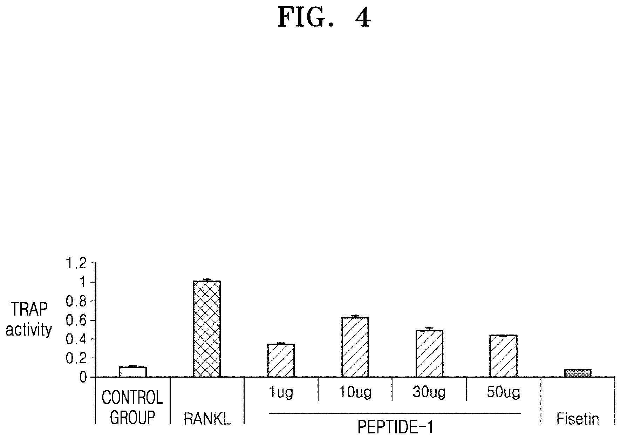

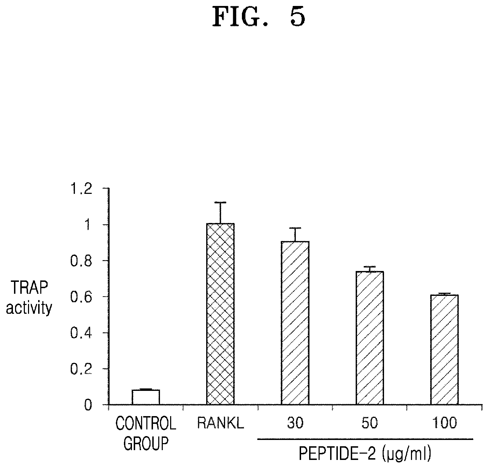

As a result, as shown in FIG. 4 and FIG. 5, it was confirmed that TRAP activity was inhibited in the group treated with Peptide-1 or Peptide-2, and this inhibitory effect showed a concentration-dependent tendency.

### Example 3. Confirmation of Effect of Inhibiting Transcription Factors on Osteoclast Differentiation

Among the various mechanisms that regulate osteoclast differentiation, Nuclear factor of activated T-cells, cytoplasmic 1 (NFATc1), a transcription factor induced by RANKL, is known to be essential for differentiation into osteoclasts. Accordingly, the aim was to determine whether treatment with Peptide-1 or Peptide-2 inhibited transcription factors involved in osteoclast differentiation during the process of inducing differentiation into osteoclasts by treating RAW264.7 cells, a precursor osteoclast cell line, with RANKL.

Specifically, RAW264.7 cells were seeded in 96-well plates at a density of 1.7 x 10³ cells/well and cultured in DMEM for 24 hours. After 24 hours, RANKL and Peptide-1 or Peptide-2 were treated at different concentrations, and differentiation into osteoclasts was induced for 4 days. After 24 or 48 hours, the medium was suctioned, cells were harvested, and RNA was isolated. After synthesizing cDNA using cDNA synthesis kit & PCR pre-mix (Intron, Korea), RT-PCR was performed using the c-Fos and NFATc1 primers shown in Table 2 below.

**[Table 2]**

| **Primer Name** | | **Sequence (5'->3')** | **SEQ ID NO:** |
|---|---|---|---|
| NFATc1 | Forward | CCTGGTGACGTGAGACTTTC | 3 |
| | Reverse | GGCTGCTTTCATGGCTAGTA | 4 |
| c-Fos | Forward | GCCAATCTGCTGAAAGAGAA | 5 |
| | Reverse | TCAGCTTCAGGGTAGGTGAA | 6 |

In addition, in the same manner as described above, differentiation into osteoclasts was induced in RAW264.7 cells cultured in DMEM for 24 hours and after 24 or 48 hours therefrom, the medium was suctioned, the cells were harvested, and lysates were prepared, and then Western blot was performed. As antibodies for detection, sc-166940 (Santa Cruz, USA) for c-Fos and sc-7294 (Santa Cruz, USA) for NFATc1 were used.

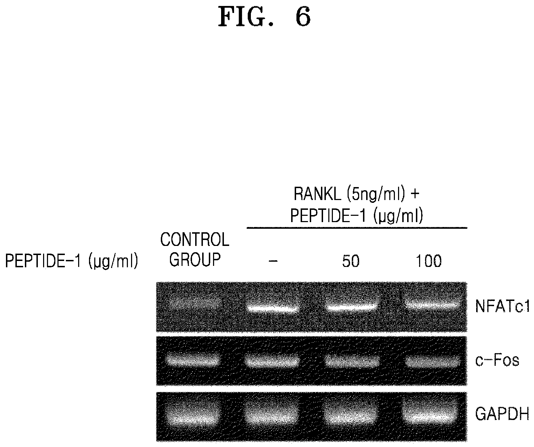

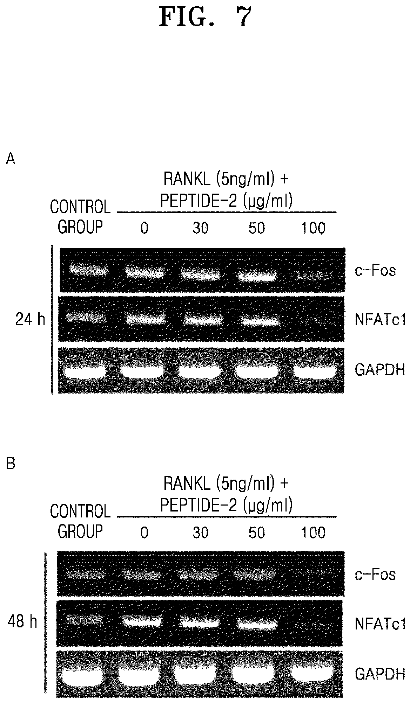

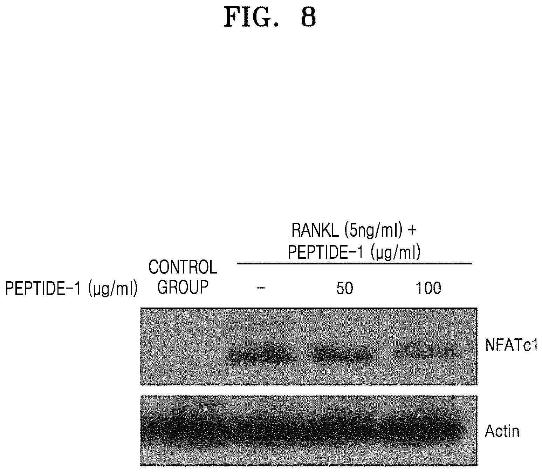

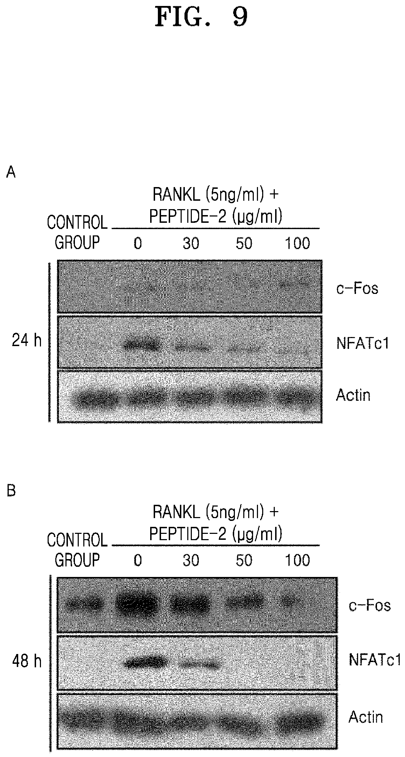

As a result, as shown in FIG. 6 to FIG. 9, it was confirmed that each of Peptide-1 and Peptide-2 inhibited the expression of osteoclast differentiation transcription factors such as NFATc1 and c-Fos induced by RANKL.

### Example 4. Confirmation of Effect of Inhibiting Downstream Signal Transducers Associated with Osteoclast Differentiation

The aim was to determine whether treatment with Peptide-1 or Peptide-2 inhibited downstream signaling molecules associated with osteoclast differentiation during the process of inducing differentiation into osteoclasts by treating RAW264.7 cells, a precursor osteoclast cell line, with RANKL.

Specifically, RAW264.7 cells were seeded in 96-well plates at a density of 1.7 x 10³ cells/well and cultured in DMEM for 24 hours. After 24 hours, RANKL and Peptide-1 or Peptide-2 were treated at different concentrations, and differentiation into osteoclasts was induced for 4 days. After 24 or 48 hours, the medium was suctioned, cells were harvested, and RNA was isolated. After synthesizing cDNA using cDNA synthesis kit & PCR pre-mix (Intron, Korea), RT-PCR was performed using the TRAP, OSCAR, CTSK, DC-STAMP, and Atp6v0d2 primers shown in Table 3 below.

**[Table 3]**

| **Primer Name** | | **Sequence (5'->3')** | **SEQ ID NO:** |
|---|---|---|---|
| TRAP | Forward | AAATCACTCTTTAAGAACAG | 7 |
| | Reverse | TTATTGAATAGCAGTGACAG | 8 |
| OSCAR | Forward | ACAGAGAAGCTGGGATCCTT | 9 |
| | Reverse | CAGCCTGAGTTCAAACCCTA | 10 |
| CTSK | Forward | GACCCATCTCTGTGTCCATC | 11 |
| | Reverse | GAAAAATGCCCTGTTGTGTC | 12 |
| DC-STAMP | Forward | GCAAGGAACCCAAGGAGTCG | 13 |
| | Reverse | CAGTTGGCCCAGAAAGAGGG | 14 |
| Atp6v0d2 | Forward | ATGGGGCCTTGCAAAAGAAA | 15 |
| | Reverse | GCTAACAACCGCAACCCCTC | 16 |
| GAPDH | Forward | ACCACAGTCCATGCCATCAC | 17 |
| | Reverse | TCCACCACCCTGTTGCTGTA | 18 |

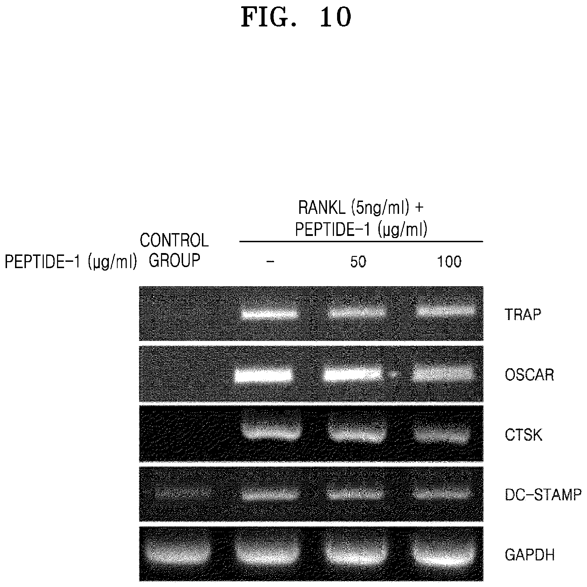

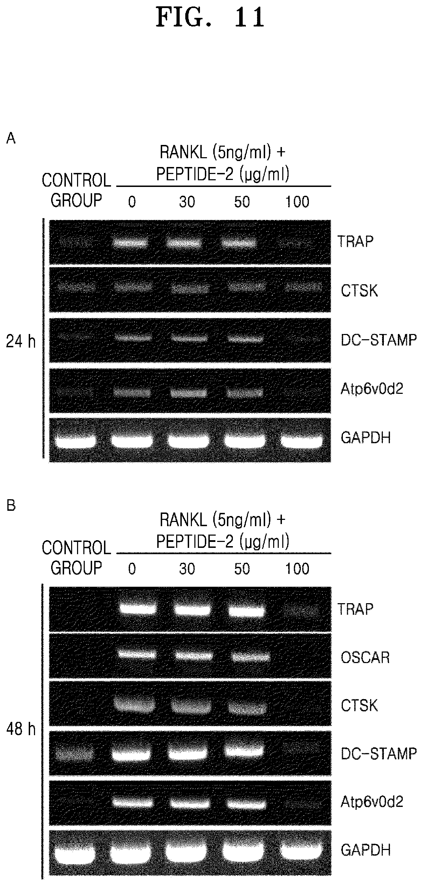

As a result, as shown in FIG. 10 and FIG. 11, it was confirmed that each of Peptide-1 and Peptide-2 inhibited the expression of downstream signal transducers associated with osteoclast differentiation, such as TRAP, OSCAR, CTSK, DC-STAMP, and Atp6v0d2d induced by RANKL.

### Example 5. Confirmation of Effect of Inhibiting Differentiation of Osteoclast Precursor Cells

The aim was to determine whether treatment with Peptide-2 inhibited the differentiation of osteoclast precursor cells in macrophages in which osteoclast differentiation was induced by RANKL.

### 5-1. Confirmation of Effect of Inhibiting Differentiation Transcription Factor

RAW264.7 cells were seeded in 96-well plates at a density of 1.7 x 10³ cells/well and cultured in DMEM for 24 hours. After 24 hours, RANKL and Peptide-2 were treated at different concentrations, and differentiation into osteoclasts was induced. After 72 hours, the medium was suctioned and treated with RANKL and Peptide-2 again, and after 3 or 6 hours, the cells were harvested and RNA was isolated. After synthesizing cDNA using cDNA synthesis kit & PCR pre-mix (Intron, Korea), RT-PCR was performed using the c-Fos and NFATc1 primers shown in Table 2 above. In addition, in the same manner as described above, differentiation into osteoclasts was induced in RAW264.7 cells cultured in DMEM for 24 hours. After 3 days, the medium was suctioned and treated with RANKL and Peptide-2 again. After 3 or 6 hours, cells were harvested, lysates were prepared, and Western blot was performed. As antibodies for detection, sc-166940 (Santa Cruz, USA) for c-Fos and sc-7294 (Santa Cruz, USA) for NFATc1 were used.

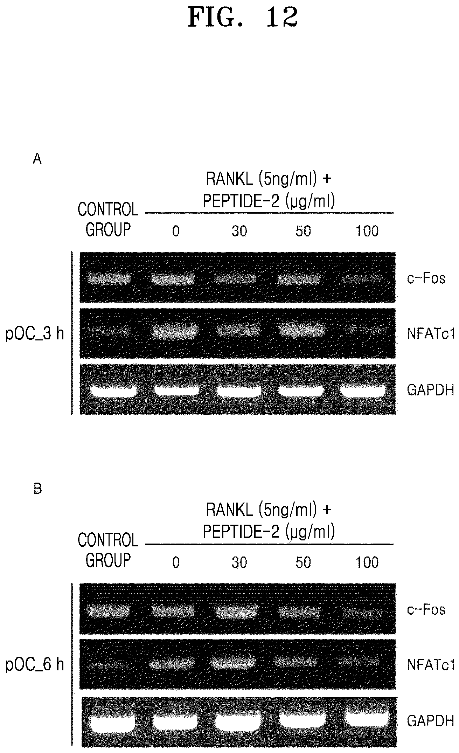

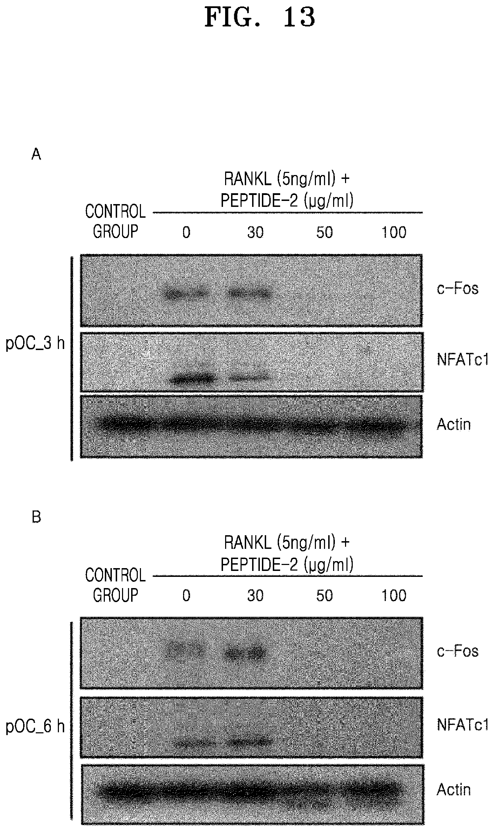

As a result, as shown in FIG. 12 and FIG. 13, it was confirmed that Peptide-2 inhibited the expression of differentiation transcription factors of osteoclast precursor cells, such as c-Fos and NFATc1.

### 5-2. Confirmation of Effect of Inhibiting Downstream Signal Transducers Associated with Differentiation

In the same manner as described above, RAW264.7 cells cultured in DMEM for 24 hours were treated with RANKL and Peptide-2 at different concentrations, and differentiation into osteoclasts was induced. After 72 hours, the medium was suctioned and treated with RANKL and Peptide-2 again. After 3 or 6 hours, cells were harvested and RNA was isolated. After synthesizing cDNA using cDNA synthesis kit & PCR pre-mix (Intron, Korea), RT-PCR was performed using the TRAP, OSCAR, CTSK, DC-STAMP, and Atp6v0d2 primers shown in Table 3 above.

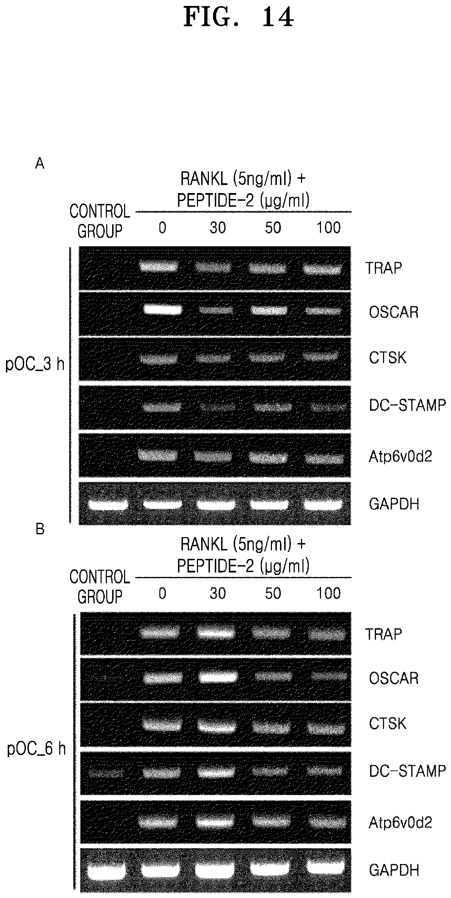

As a result, as shown in FIG. 14, it was confirmed that Peptide-2 inhibited the expression of downstream signal transducers associated with osteoclast differentiation, such as TRAP, OSCAR, CTSK, DC-STAMP, and Atp6v0d2.

In summary of the above experimental results, it was found that each of Peptide-1 and Peptide-2 according to an embodiment may be applied to the treatment of bone diseases by weakening the differentiation and/or function of osteoclasts.

### Formulation Example 1. Preparation of Peptide Nanosomes

50 mg of the peptide of Example 1 above was dissolved in 500 ml of distilled water by sufficient stirring. The composite solution was mixed with 5 g of lecithin, 0.3 ml of sodium oleate, 50 ml of ethanol, and a small amount of oil, and the total amount was adjusted to 1 L with distilled water, and the mixture was then emulsified using a mat high pressure to produce peptide nanosomes with a size of about 100 nm.

### Formulation Example 2. Pharmaceutical Preparation

### 2-1. Preparation of Powder

A powder is prepared by mixing the following ingredients and filling them into airtight pouches.
Peptide of the present disclosure 20 mg
Lactose 100 mg
Talc 10 mg

### 2-2. Preparation of Tablet

A tablet is prepared by mixing the following ingredients and compressing them according to a usual method of tablet preparation.
Peptide of the present disclosure 10 mg
Corn starch 100 mg
Lactose 100 mg
Magnesium stearate 2 mg

### 2-3. Preparation of Capsule

A capsule is prepared by mixing the following ingredients according to a usual method of capsule preparation and filling the mixture into gelatin capsules.
Peptide of the present disclosure 10 mg
Crystalline cellulose 3 mg
Lactose 14.8 mg
Magnesium stearate 0.2 mg

### 2-4. Preparation of Injection

An injection is prepared according to a usual method of injection preparation such that the following ingredients are contained per 2 ml ampoule.
Peptide of the present disclosure 10 mg
Mannitol 180 mg
Sterile distilled water for injection 2974 mg
Na₂HPO₄·2H2O 26 mg

### 2-5. Preparation of Liquid Formulation

According to the usual method of preparing a liquid, each ingredient is added to purified water and dissolved, the following ingredients are mixed, purified water is added to adjust the total to 100 ml, and the liquid is then filled into a brown bottle and sterilized to prepare the liquid.
Peptide of the present disclosure 10 mg
Isomerized sugar 10 g
Mannitol 5 g
Appropriate amount of purified water

The foregoing description of the present disclosure is for illustrative purposes only, and one that has ordinary skill in the art to which the present disclosure belongs will understand that it may be readily adapted to other specific forms without altering the technical ideas or essential features of the present disclosure. Therefore, the examples described above should be understood in all respects as illustrative and not restrictive.

## Claims

1. A peptide consisting of an amino acid sequence of SEQ ID NO: 1 or SEQ ID NO: 2.

2. The peptide of claim 1, wherein an N-terminus of the peptide is bonded to any one protecting group selected from the group consisting of an acetyl group, a fluoreonylmethoxycarbonyl group, a formyl group, a palmitoyl group, a myristyl group, a stearyl group, a butoxycarbonyl group, an allyloxycarbonyl group, and polyethylene glycol (PEG).

3. The peptide of claim 1, wherein a C-terminus of the peptide is bonded to any one protecting group selected from the group consisting of an amino group (-NH₂), a tertiary alkyl group, and azide (-NHNH₂).

4. The peptide of claim 1, wherein the peptide exhibits any one or more selected from the following characteristics:
(a) Inhibition of osteoclast differentiation;
(b) inhibition of expression of nuclear factor-activated T cells cytoplasmic 1 (NFATc1) or c-Fos;
(c) inhibition of expression of tartrate-resistant acid phosphatase (TRAP), osteoclast-associated receptor (OSCAR), cathepsin K (CTSK), or dendritic cell-specific transmembrane protein (DC-STAMP); and
(d) inhibition of expression of ATPase H+ Transporting V0 Subunit D2 (Atp6v0d2).

5. A pharmaceutical composition for preventing or treating a bone disease, comprising, as an active ingredient, a peptide according to any one of claims 1 to 4.

6. The pharmaceutical composition of claim 5, further comprising a pharmaceutically acceptable carrier.

7. The pharmaceutical composition of claim 5, wherein the peptide is formulated in nanosome form.

8. The pharmaceutical composition of claim 5, wherein the bone disease is osteoporosis, osteogenesis imperfecta, osteomalacia, osteonecrosis, rickets, osteomyelitis, alveolar bone loss, Paget's disease of bone, hypercalcemia, primary hyperparathyroidism, myeloma, bone loss in rheumatoid arthritis, bone loss due to cancer, fibrous dysplasia, aplastic bone disease, metabolic bone disease, or age-related loss of bone mass.
